# FASCICULE DE BREVET EUROPEEN

(11) **EP 0 789 542 B1**
(45) Date de publication et mention de la délivrance du brevet: **03.05.2000**
(21) Numéro de dépôt: 95930573.1
(22) Date de dépôt: 12.09.1995
(51) Int. Cl.: A61F 6/00

(54) **BOITIER DE CONDITIONNEMENT POUR PRESERVATIF**
KONDOMVERPACKUNG
CONDOM PACKAGING CASE

(30) Priorité: 12.09.1994 FR 9410852
(43) Date de publication de la demande: 20.08.1997
(73) Titulaire: Loops Company, 75016 Paris (FR)
(72) Inventeur: DE VILMORIN, Yves, 75007 Paris (FR); BAIGNERES, Frédéric, 75016 Paris (FR); ARBIN, Christian, 45100 Orléans (FR)
(74) Mandataire: Leboyer, Jean-Jacques
(86) Numéro de dépôt international: FR9501166
(87) Numéro de publication internationale: WO9608220

(56) Documents cités:
- WO-A-90/04545
- DE-U- 8 703 111
- FR-A- 381 058
- US-A- 4 892 188

## Description

La présente invention concerne un boîtier de conditionnement étanche, inviolable et non réutilisable pour préservatif.

Dans le domaine de conditionnement unitaire des préservatifs, on connaît essentiellement des sachets constitués par deux feuilles composites en regard, qui sont chacune généralement à base d'aluminium et de matière plastique et sont soudées par voie thermique autour de chaque préservatif, de préférence selon des bandes de soudage rectilignes perpendiculaires entre elles.

Ces sachets de conditionnement unitaire présentent tous un ou plusieurs des inconvénients suivants :
- leur étanchéité dépend de la pression exercée par tout objet pointu portant contre leur paroi,
- le maintien de l'intégrité du latex constituant le préservatif dépend également de la proximité de tout objet pointu, mais dépend surtout des précautions prises pour déchirer les feuilles composites et extraire le préservatif sans que ce dernier n'entre en contact avec les feuilles d'aluminium ou de métal,
- dans le cas des sachets, qui est de loin le mode de conditionnement le plus employé, le préservatif est extrait de façon aléatoire, soit dans le "bon" sens permettant de le saisir par le réservoir dénommé ci-après "chapeau" pour en chasser directement l'air, soit en sens inverse, ce qui implique une fastidieuse manipulation supplémentaire ainsi que des erreurs d'utilisation entraînant des souillures de la face externe du préservatif,
- dans le cas également des sachets, les procédés de fabrication actuels ne permettent pas de centrer la dénomination commerciale sur le produit soudé ni même de réaliser l'étiquetage légal en la matière,
- le prix de revient de certains emballages est très élevé.

Un boîtier de conditionnement pour préservatif selon le préambule de la revendication 1 est connu de FR-A-381 058.

La présente invention vise à remédier aux inconvénients précités en fournissant un boîtier de conditionnement pour préservatif, dont l'étanchéité et la solidité garantissent la parfaite conservation du préservatif en toutes circonstances, dont l'ouverture libère le préservatif dans le "bon" sens pour le saisir en en chassant l'air par la pression simple du pouce et de l'index et sans aucun risque de détérioration du latex, qui autorise le centrage de toute mention sur ses deux faces et dont le prix de revient reste faible.

Conformément à l'invention, le boîtier est du type étanche, inviolable et non réutilisable.

Selon une caractéristique essentielle de l'invention, le boîtier comprend un corps et un couvercle reliés par une articulation latérale, constitués par une matière plastique et venus ensemble de moulage, ledit corps comportant une gorge annulaire présentant un fond, une paroi extérieure à faible dépouille et une paroi intérieure à forte dépouille terminée par un bossage central de raidissement, ladite gorge annulaire étant destinée au logement du toron du préservatif et la paroi intérieure à forte dépouille étant destinée à supporter la face interne du préservatif, reliant le toron au chapeau terminal de ce dernier, ledit chapeau étant logé entre le bossage et le couvercle lorsque le boîtier est fermé.

Selon une autre caractéristique de l'invention, le corps comporte un bord périphérique saillant vers l'extérieur et une lèvre périphérique saillant au-dessus de la gorge annulaire et entourant cette dernière, le couvercle comportant un bord périphérique saillant vers l'extérieur et une rainure périphérique de même diamètre que la lèvre et destinée à coopérer avec cette dernière pour former un fin cordon de soudure périphérique étanche lors de l'opération de fermeture du boîtier, lesdits bords périphériques étant séparés par un court intervalle lorsque le boîtier est fermé.

Selon encore une autre caractéristique de l'invention le corps comporte une cuvette cylindrique délimitée par la face inférieure du bossage central et par la face intérieure de la paroi intérieure de la gorge annulaire et comprend un opercule destiné à obturer la cuvette et une seconde articulation latérale reliant l'opercule et le corps et venue de moulage avec ces derniers. De préférence, l'opercule comporte une lèvre périphérique saillant perpendiculairement à la surface locale de l'opercule et présentant sur sa face externe une faible contre-dépouille, tandis que la face périphérique intérieure de la cuvette est perpendiculaire à la surface locale du bossage et présente également une faible contre-dépouille, ces deux contre-dépouilles coopérant pour que la fermeture de la cuvette par l'opercule s'effectue avec enclenchement pour former un réservoir complémentaire étanche.

Selon une forme de réalisation préférée, le boîtier fermé se présente comme une galette circulaire à faces planes et parallèles reliées par une surface extérieure arrondie du type torique.

D'autres caractéristiques et avantages de l'invention ressortiront mieux de la description qui va suivre de plusieurs formes possibles de réalisation, faite en regard des dessins annexés sur lesquels :

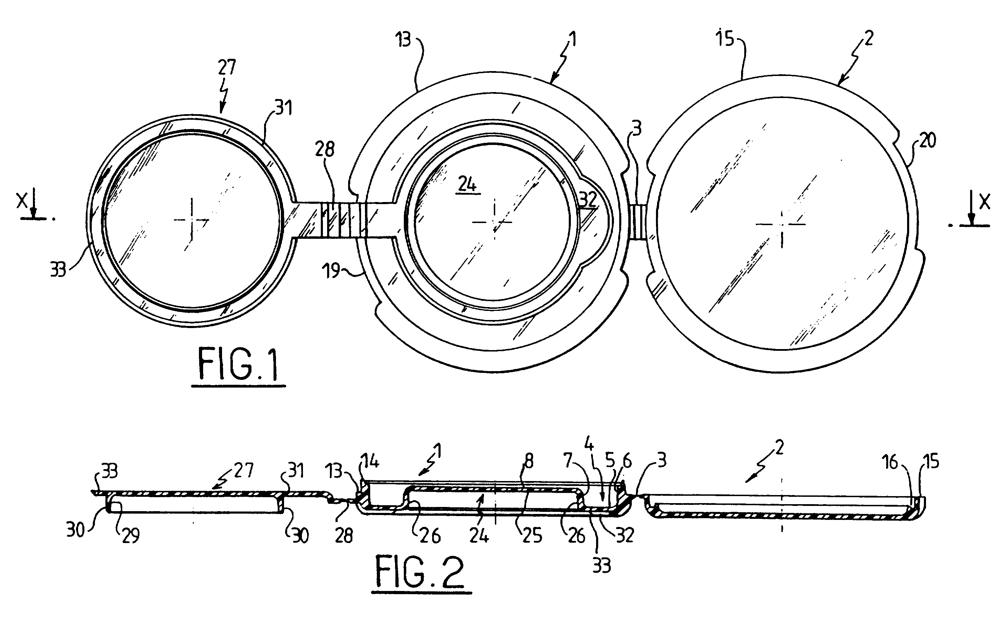

la figure 1 représente une vue de dessous d'une forme de réalisation préférée du boîtier selon l'invention venant de moulage ;
la figure 2 représente une vue en coupe suivant le plan de symétrie X-X du boîtier représenté sur la fig. 1 ;

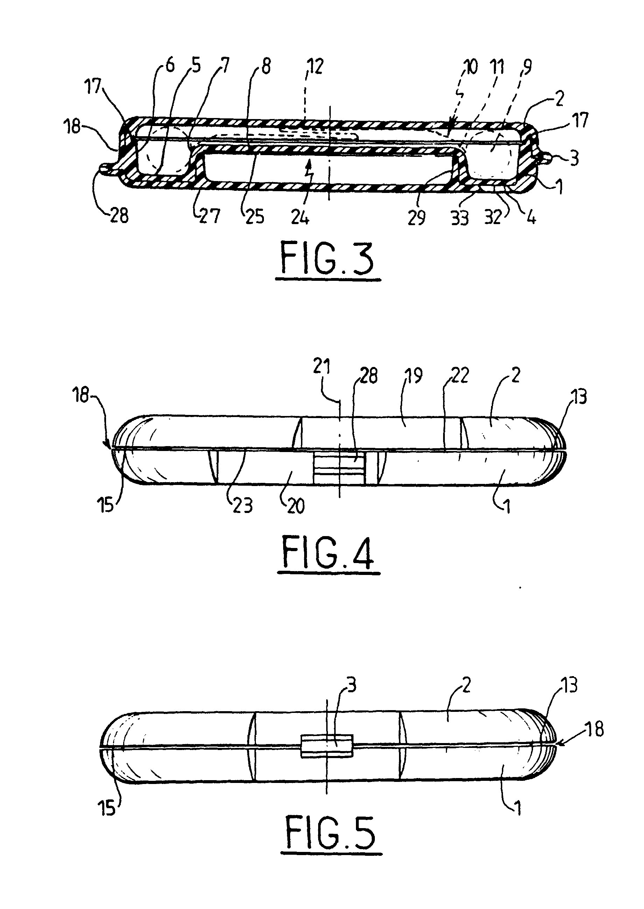

la figure 3 représente à échelle agrandie une vue en coupe selon le même axe du boîtier fermé et contenant un préservatif représenté schématiquement ;
la figure 4 représente une vue avant du boîtier fermé représenté sur la fig. 3 ; et
la figure 5 représente une vue arrière du boîtier fermé représenté sur la fig. 3.

Sur ces dsssins, les mêmes références désignent les mêmes éléments.

En se référant à l'ensemble des figures, le boîtier de conditionnement pour préservatif selon l'invention est du type étanche, inviolable et non réutilisable.

Selon une forme de réalisation préférée de l'invention, le boîtier comprend en particulier un corps 1 et un couvercle 2 reliés par une articulation latérale 3, ces trois éléments étant constitués par une matière plastique, de préférence à base de polypropylène ou de polyéthylène, et venant ensemble de moulage.

Le corps 1 comporte une gorge annulaire 4 présentant un fond 5, une paroi extérieure 6 à faible dépouille et une paroi intérieure 7 inclinée ou à forte dépouille terminée par un bossage central 8 de raidissement. La gorge annulaire 4 est destinée au logement du toron 9 d'un préservatif 10 comme cela sera expliqué plus en détail ci-après.

Selon une particularité essentielle de l'invention, la paroi conique intérieure 7 à forte dépouille est destinée à supporter la face interne 11 du préservatif, qui relie le toron 9 au chapeau terminal 12 dudit préservatif, le chapeau 12 étant logé entre le bossage 8 et le couvercle 2 lorsque le boîtier est fermé.

En fait, l'inclinaison de la paroi intérieure 7 et les dimensions de la gorge annulaire 4 sont étudiées de telle sorte que ladite paroi 7 supporte librement le toron 9 du préservatif par l'intermédiaire de sa face interne 11, ledit toron étant ainsi suspendu à flottement dans la gorge annulaire 4.

Dans ces conditions, lorsque le couvercle 2 est brutalement rabattu sur le corps 1 au cours de l'une des opérations de la chaîne de fabrication, l'air contenu dans le chapeau 12 est violemment chassé autour du bossage central 8 et son écoulement périphérique peut s'effectuer librement, d'abord entre la face interne 11 du préservatif et la paroi intérieure 7, puis entre le toron flottant 9 et le fond 5 ainsi que la paroi externe 6 de la gorge annulaire 4, sans qu'il en résulte pour ledit toron 9 aucun déplacement latéral définitif par rapport à ladite gorge annulaire 4.

Selon une autre particularité de l'invention, le corps 1 comporte un bord périphérique 13 saillant vers l'extérieur dans le prolongement dudit corps 1 et une lèvre périphérique 14 saillant au-dessus de la gorge annulaire 4 et entourant cette dernière, tandis que le couvercle 2 comporte un bord périphérique 15 saillant vers l'extérieur dans le prolongement dudit couvercle 2 et une rainure périphérique 16 de même diamètre que la lèvre 14 du corps 1. Il est clair que la lèvre 14 et la rainure associée 16 peuvent être liées de façon étanche par tout moyen de chauffage ou de collage, la rainure pouvant même être remplacée par une seconde lèvre saillant exactement en regard de la lèvre 14 pour former le réceptable étanche et inviolable destiné au préservatif.

Toutefois, selon une forme de réalisation préférée de l'invention, la rainure 16 du couvercle 2 est destinée à coopérer avec la lèvre 14 du corps 1 pour former un fin cordon de soudure périphérique étanche 17 lors de l'opération de fermeture du boîtier de manière à rendre ce dernier non seulement étanche, mais également inviolable comme indiqué ci-dessus.

Selon un mode opératoire préféré, le couvercle 2 est soumis aux vibrations provoquées par une tête à ultrasons tandis que le corps 1 reste fixe, ce qui engendre un échauffement très localisé et la formation du cordon de soudure précité 17 sans aucun risque de détérioration du latex du toron 9 situé à proximité, comme cela pourrait être le cas avec d'autres modes de soudage.

Dans le cas présent, on peut considérer que le soudage aux ultrasons est du type pointe contre pointe inverse, la rainure 16 pouvant présenter un angle rentrant d'environ 90° et la lèvre 14 un angle saillant d'environ 60°, l'ouverture de l'angle de la rainure 16 permettant un meilleur écoulement de la matière en fusion. Toutefois, les recherches qui ont conduit à l'invention ont montré qu'il était également souhaitable d'envisager sur la pointe de la lèvre 14 une petite partie latérale saillant vers l'extérieur (non représentée) et de jouer sur la flexibilité de la lèvre pour obtenir un meilleur cordon de soudure.

Il y a lieu de noter que lorsque le boîtier est soudé comme indiqué ci-dessus, les bords périphériques 13 et 15 restent séparés par un court intervalle 18. De préférence, les deux bords périphériques saillant vers l'extérieur comportent chacun une partie échancrée vers l'intérieur venue de moulage. Les parties échancrées 19 et 20 des deux bords respectifs 13 et 15 sont ménagées sensiblement à l'opposé de l'articulation latérale 3, à une même distance de l'axe de symétrie 21 du boîtier passant par ladite articulation 3 et symétriquement par rapport à cet axe 21.

De ce fait, les parties non échancrées situées en regard des parties échancrées 19 et 20 forment deux onglets séparés 22 et 23, symétriques par rapport à l'axe 21. Ces onglets constituent un moyen possible d'ouverture du boîtier en permettant de déchirer le cordon de soudure 17 et d'écarter le couvercle 2 du corps 1 à l'encontre de la résistance offerte par ledit cordon de soudure 17, rendant ainsi le boîtier non réutilisable. En effet, s'il contient un préservatif et n'est pas soudé ou fermé autrement comme indiqué, le boîtier ne peut plus se refermer normalement du fait que le latex du chapeau terminal 12 et l'air se trouvant dans ce dernier offrent une résistance élastique à toute pression de fermeture et provoquent l'écartement du couvercle 2 par rapport au corps 1 dès que cette pression n'est plus exercée.

Selon une autre particularité de l'invention, le fait d'écarter le couvercle 2 par rapport au corps 1 provoque, lors de la première ouverture du boîtier par déchirement du cordon de collage ou de soudure étanche 17, le redressement du chapeau terminal 12, permettant ainsi de saisir immédiatement ledit chapeau 12 dans le "bon" sens pour en chasser l'air avant utilisation. Par ailleurs, la partie extérieure du chapeau terminal 12 est normalement lubrifiée avant les opérations de fermeture et de soudage du boîtier, le lubrifiant pouvant être à base de silicone alimentaire par exemple.

Selon encore une autre particularité essentielle de l'invention, le corps 1 comporte une cuvette cylindrique 24 délimitée par la face inférieure 25 du bossage central 8 et par la face intérieure 26 de la paroi intérieure 7 de la gorge annulaire 4 et comprend un opercule 27 destiné à obturer la cuvette 24 et une seconde articulation latérale 28 reliant l'opercule 27 et le corps 1 et venue de moulage avec ces derniers.

L'opercule 27 comprend une lèvre périphérique 29 saillant perpendiculairement à la surface locale de l'opercule 27 et présentant sur sa face externe 30 une faible contre-dépouille, tandis que la face périphérique intérieure 26 de la cuvette 24 est perpendiculaire à la surface locale du bossage 8 et présente également une faible contre-dépouille, ces deux contre-dépouilles coopérant pour que la fermeture de la cuvette 24 par l'opercule 27 s'effectue avec enclenchement pour former un réservoir complémentaire étanche.

Ce réservoir peut contenir par exemple, soit un mince sachet hygiénique plié et destiné à recevoir le préservatif après usage, soit un gel de lubrification complémentaire, ce dernier pouvant être aromatisé ou non et coloré ou non.

Par ailleurs, l'opercule 27 comporte de préférence un mince bord périphérique 31 saillant vers l'extérieur dans le prolongement dudit opercule et dont au moins la partie opposée à la seconde articulation 28 est biseautée et coopère avec une petite zone creuse 32 ménagée sur le bord de la cuvette 24 pour former un onglet 33 d'écartement de l'opercule 27 par rapport à la cuvette 24. D'autres moyens d'ouverture du réservoir complémentaire peuvent évidemment être envisagés.

Lorsqu'il est fermé, le boîtier présente avantageusement la forme d'un ellipsoïde ou d'un hyperboloïde de révolution dans lequel le bossage 8 peut être plan, bombé ou creux.

Toutefois, selon une forme de réalisation préférée, le boîtier fermé présente simplement la forme générale d'une galette circulaire à faces planes et parallèles reliées par une surface extérieure arrondie du type torique. De préférence également, les bords périphériques 13 et 15 du corps 1 et du couvercle 2, qui sont séparés par un court intervalle 18, sont généralement plans.

En outre, le bossage 8 est de préférence plan, de sorte que la cuvette 24 forme avec l'opercule 27 un réservoir cylindrique droit.

Selon encore une autre particularité de l'invention, l'opercule 27 ne fait pas saillie par rapport à la surface du corps 1 et, par conséquent, une étiquette d'inviolabilité du réservoir peut être collée à la fois sur ladite surface et sur l'opercule. Cette étiquette comporte de préférence le mode d'emploi complet avec toutes les mentions légales en la matière.

Par contre, le couvercle 2 du boîtier peut être utilisé comme support publicitaire pour une étiquette collée ou pour une représentation obtenue par tampographie ou autrement. Outre son intérêt commercial évident, une telle publicité amène l'utilisateur à ouvrir le boîtier dans le "bon" sens, c'est-à-dire avec la publicité et donc le couvercle 2 au-dessus et avec l'étiquette du mode d'emploi et donc l'opercule 27 et le réservoir complémentaire au-dessous. Dans ces conditions, l'utilisateur n'a aucun mal à saisir le préservatif 10 par son chapeau terminal 12 pour en évacuer l'air avant usage.

Par ailleurs, un certain nombre de boîtiers selon l'invention peuvent être moulés de façon connue en grappe, puis séparés les uns des autres avec élimination des carottes de moulage et autres éléments extérieurs. En outre, les trois organes de base reliés par les deux articulations 3 et 28, c'est-à-dire le couvercle 2, le corps 1 et l'opercule 27, peuvent avoir tous une face située dans un plan, non représenté, commun à ces trois organes.

Il est bien entendu que la présente invention n'a été décrite et représentée qu'à titre explicatif mais nullement limitatif et qu'on pourra y apporter toute modification utile, notamment dans le domaine des équivalences techniques, sans sortir de son cadre.

## Revendications

1. Boîtier de conditionnement pour un préservatif, comprenant un corps (1) et un couvercle (2), le corps (1) comportant une gorge annulaire (4) présentant un fond (5), une paroi extérieure (6) et une paroi intérieure (7) terminée par un bossage central (8) de raidissement, la gorge (4) étant destinée au logement du toron (9) du préservatif (10) et la paroi intérieure (7) étant destinée à supporter la face interne (11) du préservatif, reliant le toron (9) au chapeau terminal (12) dudit préservatif, ledit chapeau (12) étant logé entre le bossage (8) et le couvercle (2) lorsque le boîtier est fermé, caractérisé par le fait que le corps (1) et le couvercle (2) sont reliés par une articulation latérale (3), et que le corps (1), le couvercle (2) et l'articulation latérale (3) sont constitués par une matière plastique et venus ensemble de moulage, la paroi extérieure (6) de la gorge annulaire (4) présentant une faible dépouille et la paroi intérieure (7) de ladite gorge (4) présentant une forte dépouille de manière à pouvoir supporter librement le toron (9) du préservatif par l'intermédiaire de sa face interne (11), ledit toron (9) pouvant ainsi être suspendu à flottement dans la gorge annulaire (4), de telle sorte que lorsque le couvercle (2) est brutalement rabattu sur le corps (1) au cours de l'une des opérations de la chaîne de fabrication, l'air contenu dans le chapeau (12) est violemment chassé autour du bossage central (8) et son écoulement périphérique peut s'effectuer librement, d'abord entre la face interne (1) du préservatif et la paroi intérieure (7) de la gorge annulaire (4), puis entre le toron flottant (9) et le fond (5) ainsi que la paroi externe (6) de la gorge annulaire (4), sans qu'il en résulte pour ledit toron (9) aucun déplacement latéral définitif par rapport à ladite gorge annulaire (4).

2. Boîtier suivant la revendication 1, caractérisé par le fait que le corps (1) comporte un bord périphérique (13) saillant vers l'extérieur dans le prolongement dudit corps et une lèvre périphérique (14) saillant au-dessus de la gorge annulaire (4) et entourant cette dernière, le couvercle (2) comportant un bord périphérique (15) saillant vers l'extérieur dans le prolongement dudit couvercle et une rainure périphérique (16) de même diamètre que la lèvre (14) et destinée à coopérer avec cette dernière pour former un fin cordon de soudure périphérique étanche (17) lors de l'opération de fermeture du boîtier, lesdits bords périphériques (13, 15) étant séparés par un court intervalle (18) lorsque le boîtier est fermé.

3. Boîtier suivant la revendication 2, caractérisé par le fait que les deux bords périphériques (13, 15) saillant vers l'extérieur comportent chacun une partie échancrée vers l'intérieur venue de moulage, les parties échancrées (19, 20) des deux bords (13, 15) étant ménagées sensiblement à l'opposé de l'articulation latérale (3), à une même distance de l'axe de symétrie (21) du boîtier passant par ladite articulation (3) et symétriquement par rapport à cet axe (21), les parties non échancrées situées en regard desdites parties échancrées (19, 20) formant deux onglets (22, 23) symétriques par rapport audit axe (21) et destinés à l'ouverture du boîtier à l'encontre du cordon de soudure (17).

4. Boîtier suivant l'une quelconque des revendications 1 à 3, dans lequel le corps (1) comporte une cuvette cylindrique (24) délimitée par la face inférieure (25) du bossage central (8) et par la face intérieure (26) de la paroi intérieure (7) de la gorge annulaire (4) et comprend un opercule (27) destiné à obturer la cuvette (24), caractérisé par le fait que le corps (1) et l'opercule (27) sont relié par une seconde articulation latérale (28) venue de moulage avec ces derniers.

5. Boîtier suivant la revendication 4, caractérisé par le fait que l'opercule (27) comporte une lèvre périphérique (29) saillant perpendiculairement à la surface locale de l'opercule (27) et présentant sur sa face externe (30) une faible contre-dépouille, tandis que la face périphérique intérieure (26) de la cuvette (24) est perpendiculaire à la surface locale du bossage (8) et présente également une faible contre-dépouille, ces deux contre-dépouilles coopérant pour que la fermeture de la cuvette (24) par l'opercule (27) s'effectue avec enclenchement pour former un réservoir complémentaire étanche.

6. Boîtier suivant l'une des revendications 4 ou 5, caractérisé par le fait que l'opercule (27) comporte un mince bord périphérique (31) saillant vers l'extérieur dans le prolongement dudit opercule, dont au moins la partie opposée à la seconde articulation (28) est biseautée et coopère avec une petite zone creuse (32) ménagée sur le bord de la cuvette (24) pour former un onglet (33) d'écartement de l'opercule (27) par rapport à la cuvette (24).

7. Boîtier suivant l'une quelconque des revendications 1 à 6, caractérisé par le fait que lorsqu'il est fermé, il présente la forme générale d'une galette circulaire à faces planes et parallèles reliées par une surface extérieure arrondie du type torique, les bords périphériques (13, 15) du corps (1) et du couvercle (2) séparés par un court intervalle (18) étant plans.

8. Boîtier suivant l'une quelconque des revendications 1 à 7, caractérisé par le fait que le bossage (8) est plan et la cuvette (24) forme avec l'opercule (27) un réservoir cylindrique droit.

9. Boîtier suivant l'une quelconque des revendications 4 à 8, caractérisé par le fait que l'opercule (27) ne fait pas saillie par rapport à la surface du corps (1) et une étiquette d'inviolabilité du réservoir peut être collée à la fois sur ladite surface et sur l'opercule.

10. Boîtier suivant l'une quelconque des revendications 1 à 9, caractérisé par le fait que le couvercle (2) du boîtier est utilisé comme support publicitaire.

## Patentansprüche

1. Kondomverpackungsbehälter, der einen Körper (1) und einen Deckel (2) umfasst, wobei der Körper (1) eine ringförmige Aussparung (4) mit einem Boden (5), einer Außenwand (6) und einer Innenwand (7), die durch einen zentralen Versteifungshöcker (8) begrenzt ist, umfasst, wobei die Aussparung (4) zum Unterbringen des Ringwulstes (9) des Kondoms (10) vorgesehen ist und die Innenwand (7) zum Stützen der Innenfläche (11) des Kondoms, die den Ringwulst (9) mit der Abschlusskappe (12) des Kondoms verbindet, vorgesehen ist, wobei die Kappe (12) zwischen dem Höcker (8) und dem Deckel (2) untergebracht ist, wenn der Behälter in geschlossener Stellung vorliegt, dadurch gekennzeichnet, dass der Körper (1) und der Deckel (2) durch ein seitliches Gelenk (3) verbunden sind, und dadurch, dass der Körper (1), der Deckel (2) und das seitliche Gelenk (3) aus einem Plastikmaterial bestehen und integral miteinander geformt sind, wobei die Außenwand (6) der ringförmigen Aussparung (4) eine geringfügige Verjüngung aufweist und die Innenwand (7) der Aussparung (4) eine starke Verjüngung aufweist, um so den Ringwulst (9) des Kondoms über seine Innenfläche (11) lose stützen zu können, wobei der Ringwulst (9) auf diese Weise schwebend in der ringförmigen Aussparung (4) gehalten werden kann, derart, dass dann, wenn der Deckel (2) während eines der Fließband-Arbeitsgänge plötzlich auf den Körper (1) heruntergelassen wird, die Luft, die in der Kappe (12) enthalten ist, heftig rund um den zentralen Höcker (8) ausgestoßen wird und ihre Randströmung frei stattfinden kann, und zwar zuerst zwischen der Innenfläche (11) des Kondoms und der Innenwand (7) der ringförmigen Aussparung (4) und sodann zwischen dem schwebenden Ringwulst (9) und dem Boden (5) sowie der Außenwand (6) der ringförmigen Aussparung (4), derart, dass sich daraus keine endgültige seitliche Verschiebung des Ringwulstes (9) in Bezug auf die ringförmige Aussparung (4) ergibt.

2. Behälter nach Anspruch 1, dadurch gekennzeichnet, dass der Körper (1) eine nach außen vorstehende Randkante (13) in der Verlängerung des Körpers umfasst sowie eine Randlippe (14), die über der ringförmigen Aussparung (4) vorsteht und diese umgibt, wobei der Deckel (2) eine nach außen vorstehende Randkante (15) in der Verlängerung des Deckels umfasst, sowie eine Randrinne (16) des gleichen Durchmessers wie die Lippe (14), wobei jene vorgesehen ist, um mit letzterer zusammenzuwirken, um eine dünne dichte Umfangsschweißnaht (17) während des Arbeitsgangs des Verschließens des Behälters auszubilden, wobei die Randkanten (13, 15) durch einen kurzen Abstand (18) getrennt sind, wenn der Behälter in geschlossenem Zustand vorliegt.

3. Behälter nach Anspruch 2, dadurch gekennzeichnet, dass jede der zwei nach außen vorstehenden Randkanten (13, 15) einen integral geformten Teil aufweisen, der nach innen hin ausgeschnitten ist, wobei die ausgeschnittenen Teile (19, 20) der zwei Kanten (13, 15) im wesentlichen gegenüber dem seitlichen Gelenk (3) im gleichen Abstand von der Symmetrieachse (21) des Behälters, die durch das Gelenk (3) läuft, und symmetrisch hinsichtlich der Achse (21) angeordnet sind, wobei die gegenüber der ausgeschnittenen Teile (19, 20) gelegenen nicht-ausgeschnittenen Teile zwei Gehrungen (22, 23) bilden, die symmetrisch hinsichtlich der Achse (21) sind und zum Öffnen des Behälters entgegen dem Widerstand der Schweißnaht (17) vorgesehen sind.

4. Behälter nach einem der Ansprüche 1 bis 3, wobei der Körper (1) eine zylindrische Einsenkung (24) umfasst, die durch die untere Fläche (25) des zentralen Höckers (8) und durch die Innenfläche (26) der Innenwand (7) der ringförmigen Aussparung (4) abgegrenzt ist, sowie einen Deckel (27) umfasst, der vorgesehen ist, um die Einsenkung (24) zu schließen, dadurch gekennzeichnet, dass der Körper (1) und der Deckel (27) durch ein zweites seitliches Gelenk (28) verbunden und integral mit letzterem geformt sind.

5. Behälter nach Anspruch 4, dadurch gekennzeichnet, dass der Deckel (27) eine Randlippe (29) umfasst, die senkrecht zu der lokalen Oberfläche des Deckels (27) vorsteht und eine geringfügige Hinterschneidung auf ihrer Außenfläche (30) aufweist, wobei die innere Randoberfläche (26) der Einsenkung (24) senkrecht zur lokalen Oberfläche des Höckers (8) vorliegt und auch eine geringfügige Hinterschneidung aufweist, wobei diese zwei Hinterschneidungen zusammenwirken, damit das Verschließen der Einsenkung (24) durch den Deckel (27) mit festem Ineinandergreifen stattfindet, um auf diese Weise ein dichtes ergänzendes Reservoir zu bilden.

6. Behälter nach einem der Ansprüche 4 oder 5, dadurch gekennzeichnet, dass der Deckel (27) eine dünne Randkante (31) umfasst, die nach außen in der Verlängerung des Deckels vorsteht, und von der wenigstens der Teil gegenüber des zweiten Gelenks (28) abgeschrägt ist und mit einem schmalen konkaven Bereich (32) zusammenwirkt, der an der Kante der Einsenkung (24) angeordnet ist, um eine Gehrung (33) für die Trennung des Deckels (27) von der Einsenkung (24) zu bilden.

7. Behälter nach einem der Ansprüche 1 bis 6, dadurch gekennzeichnet, dass er dann, wenn er geschlossen ist, die im wesentlichen die Form einer kreisförmigen Scheibe mit Flächen aufweist, die eben und parallel und durch eine abgerundete, ringwulstartige Außenfläche verbunden sind, wobei die durch einen kurzen Abstand (18) getrennten Randkanten (13, 15) des Körpers (1) und des Deckels (2) eben sind.

8. Behälter nach einem der Ansprüche 1 bis 7, dadurch gekennzeichnet, dass der Höcker (8) eben ist und die Einsenkung (24) mit dem Deckel (27) ein vertikales zylindrisches Reservoir bildet.

9. Behälter nach einem der Ansprüche 4 bis 8, dadurch gekennzeichnet, dass der Deckel (27) nicht von der Oberfläche der Körpers (1) vorsteht, und ein Etikett, das die Unversehrtheit des Reservoirs anzeigt, kann sowohl auf die Oberfläche als auch auf den Deckel geklebt werden.

10. Behälter nach einem der Ansprüche 1 bis 9, dadurch gekennzeichnet, dass der Deckel (2) des Behälters als ein Werbemedium verwendet wird.

## Claims

1. Condom packaging case comprising a body (1) and a cover (2), the body(1) comprising an annular recess (4) having a base (5), an outer wall (6) and an inner wall (7) terminated by a central stiffening bossing (8), the recess (4) being designed for accommodating the torus (9) of the condom (10) and the inner wall (7) being designed to support the inner surface (11) of the condom connecting the torus (9) with the terminal cap (12) of said condom, said cap (12) being accommodated between the bossing (8) and the cover (2) when the case is in the closed position, characterized in that the body (1) and the cover (2) are connected by a lateral joint (3), and that the body (1), the cover (2) and the lateral joint (3) consist of a plastic material and are integrally molded together, the outer wall (6) of the annular recess (4) having a slight taper and the outer wall (7) of said recess (4) having a high taper so as to be able to freely support the torus (9) of the condom via its inner surface (11), said torus (9) thus being able to be floatingly suspended in the annular recess (4) in such a way that when the cover (2) is abruptly lowered onto the body (1) during one of the production line operations, the air contained in the cap (12) is violently expelled around the central bossing (8), and its peripheral flow may take place freely, first between the inner surface (11) of the condom and the inner wall (7) of the annular recess (4) and then between the floating torus (9) and the base (5) as well as the outer wall (6) of the annular recess (4), in such a way that there results no definitive lateral displacement of said torus (9) with respect to said annular recess (4).

2. Case according to claim 1, characterized in that the body (1) comprises an outwardly projecting peripheral edge (13) in the prolongation of said body and a peripheral lip (14) projecting above the annular recess (4) and surrounding the latter, the cover (2) comprising an outwardly projecting peripheral edge (15) in the prolongation of said cover and a peripheral groove (16) of the same diameter as the lip (14) and designed to cooperate with the latter to form a thin tight peripheral weld seam (17) during the operation of closing the case, said peripheral edges (13, 15) being separated by a short distance (18) when the case is in the closed position.

3. Case according to Claim 2, characterized in that each of the two outwardly projecting peripheral edges (13, 15) has an integrally molded part undercut toward the interior, the undercut parts (19, 20) of the two edges (13, 15) being arranged substantially opposite the lateral joint (3) at the same distance from the axis of symmetry (21) of the case passing through said joint (3) and symmetrically with respect to said axis (21), the non-undercut parts situated opposite said undercut parts (19, 20) forming two miters (22, 23) symmetrical with respect to said axis (21) and designed for opening the case against the opposition of the weld seam (17).

4. Case according to any one of claims 1 to 3, wherein the body (1) comprises a cylindrical basin (24) delimited by the lower face (25) of the central bossing (8) and by the inner surface (26) of the inner wall (7) of the annular recess (4) and comprises a lid (27) designed to close the basin (24), characterized in that the body (1) and the lid (27) are connected by a second lateral joint (28) integrally molded with the latter.

5. Case according to claim 4, characterized in that the lid (27) comprises a peripheral lip (29) projecting perpendicularly to the local surface of the lid (27) and having a slight undercut on its outer surface (30), while the inner peripheral surface (26) of the basin (24) is perpendicular to the local surface of the bossing (8) and also has a slight undercut, these two undercuts cooperating in order that the closing of the basin (24) by the lid (27) takes place with firm interlocking so as to form a tight complementary reservoir.

6. Case according to one of claims 4 or 5, characterized in that the lid (27) comprises a thin peripheral edge (31) outwardly projecting in the prolongation of said lid, and of which at least the part opposite the second joint (28) is bevelled and cooperates with a small concave zone (32) arranged on the edge of the basin (24) to form a miter (33) of separation of the lid (27) from the basin (24).

7. Case according to any one of claims 1 to 6, characterized in that when closed, it has the general shape of a circular disk having faces that are plane and parallel and connected by a rounded external surface of the toric type, the peripheral edges (13, 15) of the body (1) and the cover (2) separated by a short distance (18) being plane.

8. Case according to any one of claims 1 to 7, characterized in that the bossing (8) is plane and the basin (24) forms with the lid (27) a right cylindrical reservoir.

9. Case according to any one of claims 4 to 8, characterized in that the lid (27) does not project from the surface of the body (1), and a label indicating tamper resistance of the reservoir may be stuck both on said surface and on the lid.

10. Case according to any one of claims I to 9, characterized in that the cover (2) of the case is used as an advertising medium.
